(19) Europäisches Patentamt
European Patent Office
Office européen des brevets

(11) Publication number: 0 017 703 A1

(12) EUROPEAN PATENT APPLICATION

(21) Application number: 80100398.9

(22) Date of filing: 25.01.80

(51) Int. Cl.³: C 07 C 121/70, C 07 C 120/00, C 08 F 222/32

(30) Priority: 26.01.79 US 6787
01.03.79 US 16134
20.03.79 US 22370
17.04.79 US 30753
03.05.79 US 35648
16.05.79 US 39561

(43) Date of publication of application: 29.10.80 Bulletin 80/22

(84) Designated Contracting States: CH DE FR GB IT

(71) Applicant: GAF CORPORATION, 140 West 51st Street, New York New York 10020 (US)

(72) Inventor: Lorenz, Donald H., 12 Radel Place, Basking Ridge New Jersey 07420 (US)
Inventor: Gruber, Bruce A., 2082 Sawbury Boulevard, Worthington Ohio 43085 (US)

(74) Representative: Schmied-Kowarzik, Volker, Dr. et al, Patentanwälte Dipl.-Ing. P. Wirth Dr. V. Schmied-Kowarzik Dipl.-Ing. G. Dannenberg, Dr. P. Weinhold Dr. D. Gudel, Dipl.-Ing. S. Schubert, Siegfriedstrasse 8 D-8000 München 40 (DE)

(54) **Copolymerizable (2-cyano-3,3-diarylacryloxy)alkylene compounds, a method of making them and the use of these compounds as ultraviolet light absorbers.**

(57) This invention relates to copolymerizable ultraviolet light absorber compounds having the formula:

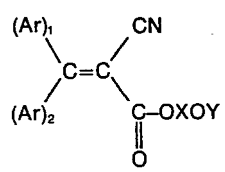

where $(Ar)_1$ and $(Ar)_2$ are aromatic carbocyclic nuclei of the benzene and naphthalene series and are independently selected from phenyl or phenyl substituted with alkyl, halo, alkoxy, carboxy, carbalkoxy, cyano, acetyl, benzoyl, phenyl, alkyl phenyl, phenoxy phenyl, alkyl substituted phenoxy, or alkoxy phenyl substituted phenyl and naphthyl;

X is alkylene, $C_2$–$C_{17}$, unsubstituted or substituted with halo, cyano, alkyl, alkoxy, alkoxyalkyl or alkoxyalkyleneoxy, $C_1$–$C_6$: and,

Y is a copolymerizable radical selected from acryloyl, alkylacryloyl, acryloxyalkyl, acryloxyhydroxyalkyl and alkylacryloxyhydroxyalkyl, $C_3$–$C_{12}$; or –RCR=CHR″, where R is alkylene, oxyalkylene, alkyleneoxyalkylene, phenylene, $C_1$–$C_{10}$, unsubstituted or substituted with hydroxy and R′ and R″ are independently hydrogen or alkyl, $C_1$–$C_{10}$.

In other aspects of the invention, it relates to methods of making these compounds and to intermediates formed during their synthesis.

EP 0 017 703 A1

This invention relates to novel copolymerizable ultraviolet light absorber compounds, and, more particularly, to (2-cyano-3,3-diphenylacryloxy) alkylene compounds which are copolymerizable with vinyl monomers to provide polymer materials having improved resistance to degradation to light.

Various organic compounds exhibit the power to absorb electromagnetic radiation and can be incorporated in various plastic materials such as transparent sheets which act as filters for all the radiation passing through and will transmit only such radiations as are not absorbed by the sheet and/or the absorbing agent. Such filters find use in many technical and commercial applications.

Numerous cyano acrylic compounds have been suggested as absorbents for the range of radiations described above. For specific compounds, see U.S. Patents 3,081,280; 3,272,810; 3,644,466; 3,256,312 and 3,215,724. These ultra-violet absorbers are mechanically mixed with the plastic materials to prevent discoloration and degradation of the material. However, it has been observed that such absorbers sometimes fail or are blocked out of the plastic under adverse weather conditions before the lifetime of the protected material. Also, it is not possible to use all of these ultra-violet absorbers with radiation curing of the plastic material. Another disadvantage of these ultra-violet absorbers is the high amount of absorber needed for protection of some materials.

What is provided herein are improved, novel compolymerizable ultra-violet light absorber compounds of the formula:

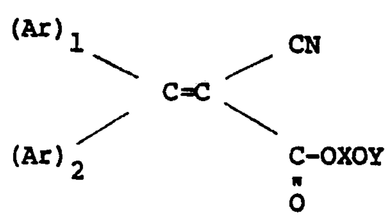

where $(Ar)_1$ and $(Ar)_2$ are aromatic carbocyclic nuclei of the benzene and naphthalene series and are independently selected from phenyl or phenyl substituted with alkyl, halo, alkoxy, carboxy, carbalkoxy, cyano, acetyl, benzoyl, phenyl, alkyl phenyl, phenoxy phenyl, alkyl substituted phenoxy, or alkoxy phenyl substituted phenyl and naphthyl;

X is alkylene, $C_2$-$C_{17}$, unsubstituted or substituted with halo, cyano, alkyl, alkoxy, alkoxyalkyl or alkoxyalkyleneoxy, $C_1$-$C_6$;

Y is a copolymerizable radical selected from acryloyl, alkylacryloyl, acryloxyalkyl, acryloxyhydroxyalkyl and alkylacryloxyhydroxyalkyl, $C_3$-$C_{12}$; or -RC'=CHR", where R is alkylene, oxyalkylene, alkyleneoxyalkylene, phenylene, $C_1$-$C_{10}$; instituted or substituted with hydroxy, and R' and R" are independently hydrogen or alkyl, $C_1$-$C_6$.

In other aspects of the invention, it relates to methods of making these compounds and to intermediates formed during their synthesis.

Suitable $(Ar)_1$ and $(Ar)_2$ groups are given in U. S. 3,644,466, including representative starting benzophenone compounds.

The X groups are unsubstituted or substituted alkylene radicals, $C_2$-$C_{17}$. The preferred groups are unsubstituted lower alkylene, $C_2$-$C_6$, which are derived synthetically from ethylene glycol, propylene glycol, butanediol, and the like.

The Y radical is compolymerizable with vinyl monomers so that the ultraviolet absorber becomes an integral part of the polymer. Suitable Y groups are derived from acryloyl, alkylacryloyl, acryloxyalkyl, acryloxyhydroxyalkyl and alkylacryloxyhydroxyalkyl, $C_3$-$C_{12}$. These groups include acryloyl, methacryloyl, glycidyl acryloyl and glycidyl methacryloxyl, 3-acryloxy-2-hydroxypropyl and 3-methacryloxy-2-hydroxypropyl.

Other Y groups include -RCR'=CHR", where R is alkylene, oxyalkylene, alkyleneoxyalkylene, phenylene, $C_1$-$C_{10}$, unsubstituted or substituted with hydroxy, and R' and R" are independently hydrogen

or alkyl, $C_1$-$C_{10}$. Suitable radicals include allyl, methylpropenyl, crotyl, ethylpropenyl, vinylbenzyl, vinyloxyethyl, allyloxy-2-hydroxypropyl and 2-hydroxy-3-butenyl.

The compounds of the invention contain ultraviolet light absorber and copolymerizable portions in the same molecule. These portions are effectively separated by the X radical so that each can perform its own function without interference from the other. Therefore, the absorber portion does not inhibit the copolymerization, and the Y radical does not affect the light absorbing properties of the molecule.

The novel compounds of the invention may be prepared by esterification of 2-hydroxyalkyl(2-cyano-3,3-diphenyl) acrylate with an acyrloyl halide or acrylic acid (Method 1) or by direct Knoevenagel condensation of a benzophenone with an acryloxyalkyl cyanoacetate (Method 2), or alkylation with an ethylenic halide.

In Method 1, the hydroxy group of a hydroxyalkyl cyano acetate first is protected by acylation with a group convertible by hydrolysis to the hydroxy compound, e.g. with acetyl chloride, to provide the corresponding acetoxyalkyl cyanoacetate. The protected compound then is condensed with a benzophenone in a Knoevenagel raction to provide the acetoxyalkyl(2-cyano-3,3-diphenyl) acrylate in good yield. Subsequent acid hydrolysis of the protecting acetyl group affords the corresponding hydroxy intermediate, which is then directly esterified with a suitable acryloyl halide or acrylic acid to give the desired compounds.

This method is summarized below:

FDN-1157 (Combined)

a) 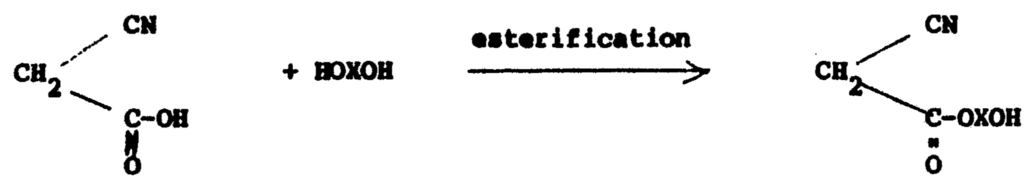


b) 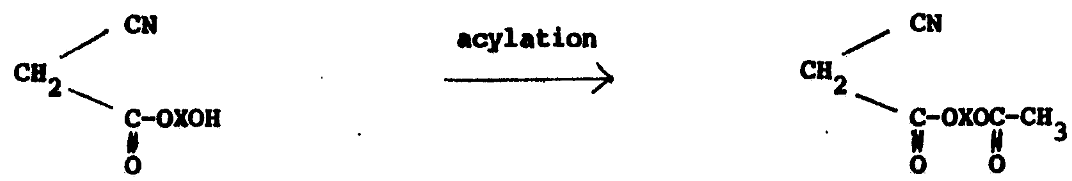

FDN-1157 (Combined)

Method 1 (Continued)

c)

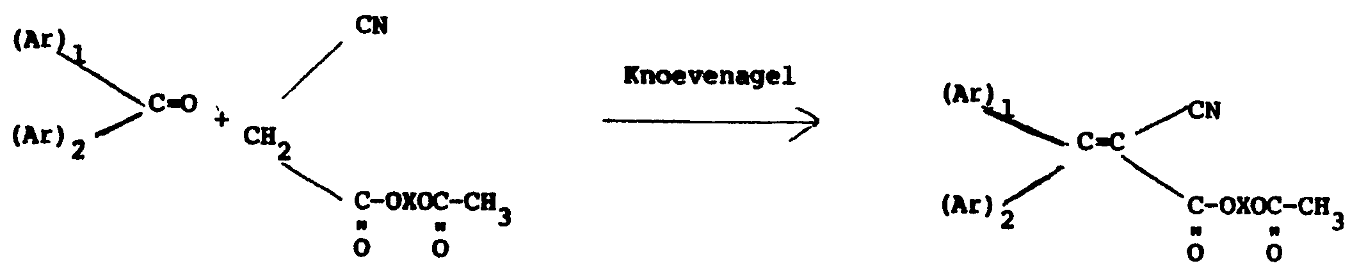


d)

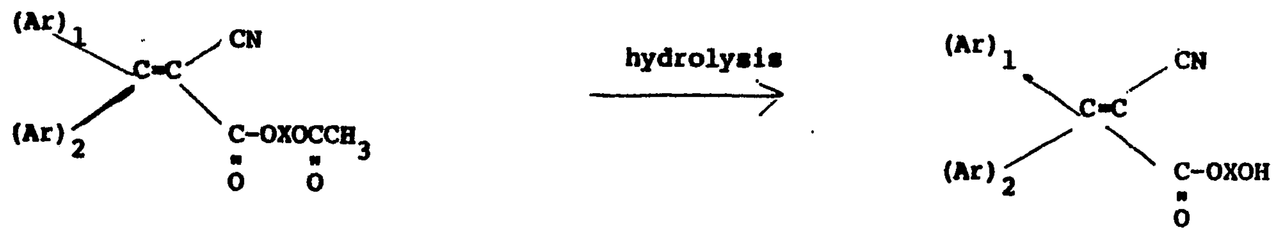

FDN-1157 (Combined)

Method 1 (Continued)

e)

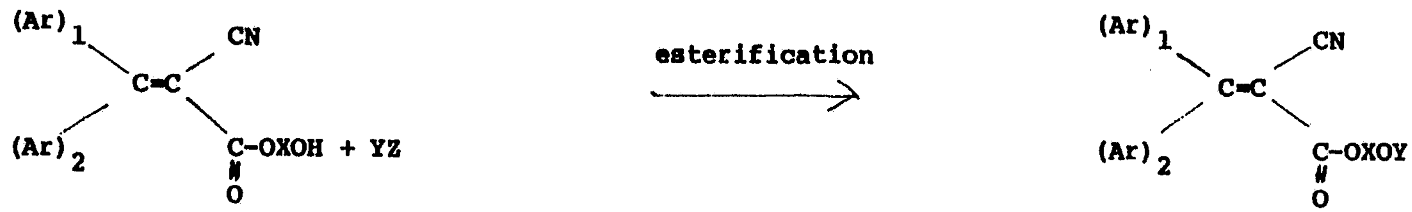


OR

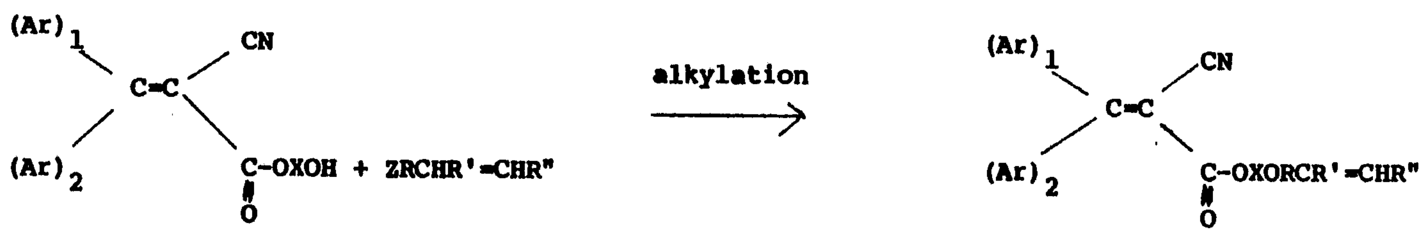

Method 1 (Continued)

where Z is a halide or hydroxyl, and X and Y and R, R' and R" are as defined above.

Typical X groups are $-CH_2CH_2$, $-CH_2CH_2CH_2-$, $CH_2CH_2CH_2CH_2-$, and the like.

Representative Y groups are $-\underset{\underset{O}{\|}}{C}-CH=CH_2$ (acryloyl), $-\underset{\underset{O}{\|}}{C}-C(CH_3)=CH_2$ (methacryloyl), $-CH_2-CH(OH)CH_2O\underset{\underset{O}{\|}}{C}CH=CH_2$ (3-acryloxy-2-hydroxypropyl), and $-CH_2CH(OH)CH_2O\underset{\underset{O}{\|}}{C}C(CH_3)=CH_2$ (3-methacryloxy-2-hydroxypropyl).

Representative $-RCR'=CHR"$ radicals are $-CH_2-CH=CH_2$ (allyl), $-CH=CHCH_3$ (crotyl), $-CH_3\underset{\underset{CH_3}{|}}{C}=CH_2$ (2-methyl-1-propenyl), 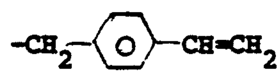 (vinylbenzyl), $-CH_2CH_2OCH=CH_2$ (vinyloxyethyl), $CH_2\underset{\underset{OH}{|}}{C}HCH_2OCH_2CH=CH_2$, (3-allyloxy-2-hydroxypropyl), and $CH_2\underset{\underset{OH}{|}}{C}HCH=CH_2$ (2-hydroxy-3-butenyl).

In step (a) of Method 1, cyanoacetic acid is reacted with a lower dihydric alcohol to form a hydroxyalkyl cyanoacetate, as described in U.S. 3,644,466, Example 3, cols. 7-8.

In step (b), the hydroxy group of the hydroxyalkyl cyanoacetate then is protected by acylation, suitable with acetic anhydride, to give the corresponding acetoxyalkyl cyanoacetate.

Step (c) in the process involves a Knoevenagel condensation of a benzophenone with the aceotxyalkyl cyanoacetate. The Knoevenagel reaction is generally run in the presence of a solvent, such as benzene, toluene, or ethylenedichloride, under reflux, usually at a temperature between 80° and 100°C. for about 24 hours. The reaction preferably proceeds in a nitrogen atmosphere and in the presence of glacial acetic acid and ammonium acetate as a catalyst. Conventional washings of the product with water and saturated bicarbonate solution are done prior to the drying, removing the solvent and recovering the product.

The fourth step (d) in the synthesis is to remove the protecting acetyl group by acid hydrolysis in alcohol to provide the corresponding free hydroxyalkyl compound. This step is carried out in methanol under acid conditions at reflux temperatures.

The final step (e) in Method 1 may involve esterification with a reactive acryloyl compound, such as an acryloyl halide e.g. acryloyl chloride or acryloyl bromide. The reaction is carried out in an inert solvent, suitably an aromatic or aliphatic hydrocarbon or halogenated hydrocarbon, such as toluene, benzene, chloroform or ethylene dichloride, or in acetone, at a suitable temperature, generally ranging from room temperature to the reflux temperature of the solvent, e.g. if chloroform, at about 61°C., and in the presence of a base, such as sodium bicarbonate, to absorb the acid by-product of the reaction. Suitably the molar ratios of the reactants are controlled to provide at least a 1:1 molar ratio of the acryloyl halide to the hydroxyalkyl(2-cyano-3,3-diphenyl) acrylate. Preferably an excess of the acryloyl chloride is used. The reaction is run for about 1-5 hours at the reflux temperature.

The yield of product in step (c) in Method 1 is about 80-90%.

An acrylic acid may be used in place of an acryloyl chloride in step (e). In this embodiment, water is distilled out of the reaction mixture as an azeotrope with the solvent. Preferably, an inhibitor, such as phenothiazine or methoxyphenol, is included in the reaction mixture in an amount of about 200-1000 ppm to prevent polymerization of the acrylic acid reactant. The reaction with acrylic acid generally is run at a somewhat higher temperature than with the acryloyl chloride, usually at about 80°-110°C, for about 10 to about 10 to about 20 hours. The yields are about 60-70%.

The final step(e) may involve alkylation with a reactive ethylenic compound, such as an ethylenic halide e.g. allyl chloride or allyl bromide. The reaction is carried out in an inert solvent, suitably an aromatic or aliphatic hydrocarbon or ether hydrocarbon, such as, toluene, benzene, tetrahydrofuran, dioxane or ethylene dichloride, at a suitable temperature, generally ranging from room temperature to the reflux temperature of the solvent, e.g. if tetrahydrofuran, at about 66°C, and in the presence of a base, such as sodium hydride, the reactants are contolled to provide at least a 1:1 molar ratio of the ethylenic halide to the hydroxyalkyl 2-cyano-3,3-diphenyl acrylate. The reaction is run for about 1-6 hours at the reflux temperature.

In Method 2, a hydroxyalkyl cyanoacetate is reacted first with an acryloyl chloride or acrylic acid to form a 2-acryloxyalkyl 2-cyanoacetate intermediate. Then the intermediate is condensed directly with a benzophenone in the Knoevenagel reaction to form the desired compounds, as follows:

<u>Method 2</u>

a) 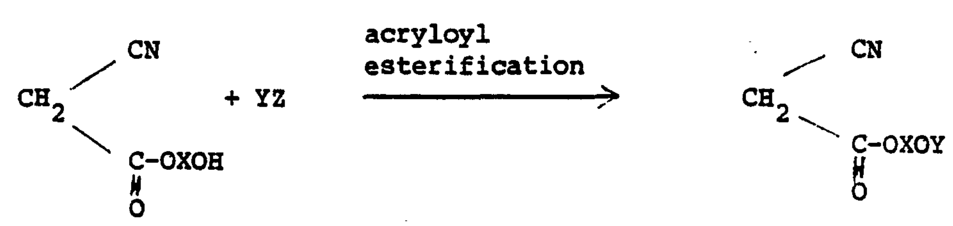

where Y may be the group -RCR'=CHR".

b)

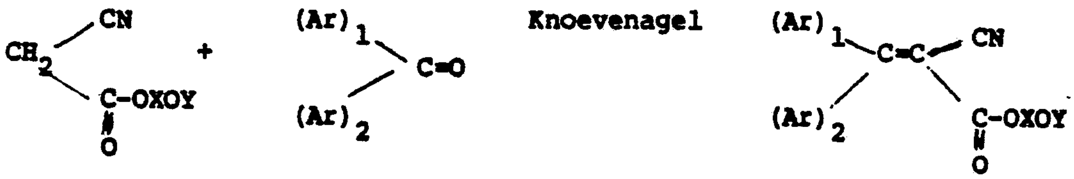


where X, Y Z and R, R'and R" are as defined above.

The reaction conditions in steps (a) and (b) of Method 2 comrrespond generally to those described in steps (e) and (c) of Method 1, respectively.

The compounds of the invention may be copolymerized, for example, with a urethane oligomer, by radiation curing, to provide useful polymeric coatings.

The following examples will describe the invention with more particularity.

## EXAMPLE 1

## 2-(2-Cyano-3,3-Diphenylacryloxy) Ethyl Acrylate

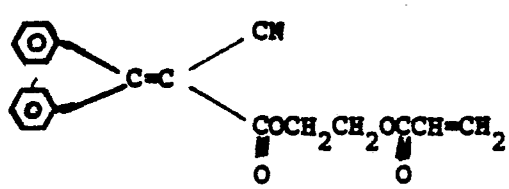

(a) 2-Hydroxyethyl 2-Cyanoacetate

Cyanoacetic acid was esterified with ethylene glycol according to U.S. Patent 3,644,466 (Col. 7-8, Ex. 3) to give the product in 74% yield.

(b) <u>2-Acetoxyethyl 2-Cyanoacetate</u>

Into a three-neck round bottom flask with magnetic stirrer, dropping funnel, thermometer, and drying tube was charged 122 g. (1.2 moles) of acetic anhydride and 10 drops of concentrated sulfuric acid. Then 129 g. (1 mole) of 2-hydroxyethyl cyanoacetate was added dropwise with stirring while maintaining the reaction temperature below 75°C. The acylated ester thus produced was then diluted with 100 ml. of water and the excess acid was neutralized with solid potassium carbonate. The oil layer was separated and dried to yield 130 g. (79%) of the desired compound.

(c) <u>2-Acetoxyethyl 2-Cyano-3,3-Diphenylacrylate</u>

A three-neck round bottom flask fitted with a mechanical stirrer, a thermometer and a Dean-Stark trap fitted with a reflux condenser was charged with 200 ml. of toluene, 182 g. (1 mole) of benzophenone, 205 g. (1.2 moles) of 2-acetoxyethyl 2-cyanoacetate, 40 ml. of glacial acetic acid, 16 g. of ammonium acetate. The contents were heated to reflux (110°C.) for 24 hours while the theoretical amount of water by-product was removed by azeotropic distillation. Upon removal of the solvent, as well as unreacted starting material by vacuum distillation, a yield of 200 g. (60%) of the desired product was obtained.

(d) <u>2-Hydroxyethyl 2-Cyano-3,3-Diphenylacrylate</u>

A charge of 800 ml. of methanol, 335 g. (1 mole) of 2-acetoxyethyl 2-cyano-3,3-diphenylacrylate and 10 drops of concentrated hydrochloric acid was heated at 65°C for 18 hours. Evaporation of the solvent left 235 g. (80%) of the intermediate compound as an amber, viscous oil.

(e) 2-(2-Cyano-3,3-Diphenylacrylate)Ethyl Acrylate

To a charge of 3 l. of methylene chloride, 179 g. of potassium carbonate and 293 g. (1 mole) of 2-hydroxyethyl 2-cyano-3,3-diphenylacrylate was added 118 g. (1.3 moles) of acryloyl chloride and the contents were heated at 41°C for 2 hours. The reaction mixture then was diluted with 3 l. of water and neutralized with solid potassium carbonate. The organic layer was separated, dried and evaporated, leaving 274 g. (79%) of an amber oil, which was characterized by NMR as the product compound.

## EXAMPLE 2

The compound of Example 1 was prepared using acrylic acid instead of acryloyl chloride in Step (e) of Example 1 as follows:

(e) A charge of 347 g. (1 mole) of 2-hydroxyethyl 2-cyano-3,3-diphenylacrylate in 1.5 l. of toluene was heated at reflux for 16 hours. The reaction mixture then was cooled to room temperature and 1 l. of water was added and the solution was neutralized with solid potassium carbonate. the organic layer then was dried and evaporated to yield 328 g. (80% yield, about 75% purity) of an amber oil of the product compound.

## EXAMPLE 3

2-(2-Cyano-3,3-diphenylacryloxy) Ethyl Methacrylate

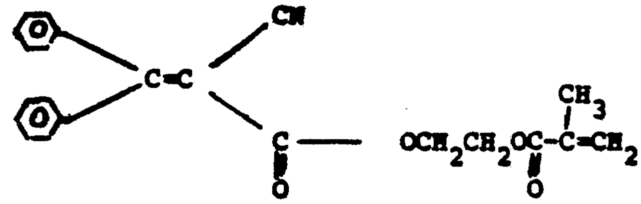

Using an equivalent amount of methacryloyl chloride in place of acryloyl chloride in Step (e) of Example 1, the desired ethyl methacrylate compound is obtained in comparable yield.

EXAMPLE 4

3-(2-Cyano-3,3-Diphenylacryloyl) Propyl Methacrylate

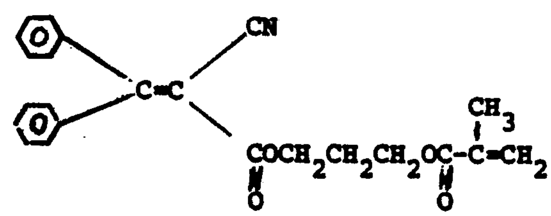

Using an equivalent amount of propylene glycol in place of ethylene glycol in Step (a) in Example 3, there is produced the desired propylmethacrylate compound in comparable yield.

EXAMPLE 5

4-(2-Cyano-3,3-Diphenylacryloyl) Butyl Acrylate

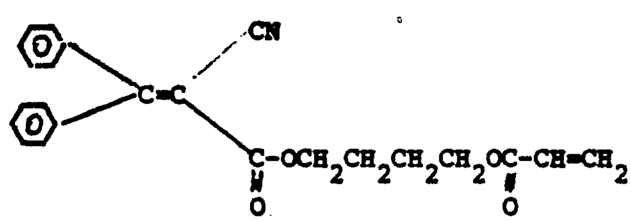

By substituting 1,4-butanediol in place of ethylene glycol in Step (a) of Example 1, there is produced the corresponding butyl acrylate.

## EXAMPLE 6

### 2-Hydroxy-3- 2-(2-Cyano-3,3-Diphenyl) Ethyoxy Propyl Acrylate

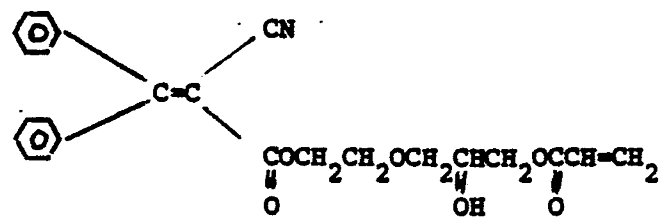

The procedure of Example 1 is followed except for (e) as follows:

Into a round bottom flask is charged 29.3 g. (0.1 mole) of 2-hydroxyethyl 2-cyano-3,3-diphenylacrylate, 21.3 g. (0.15 mole) glycidyl acrylate and 0.27 g. (0.0025 mole) tetramethylammonium chloride. The mixture was heated at 70-90$^{\circ}$ for 5 hours. Thereafter excess glycidyl acrylate was removed by vacuum distillation. The remaining amber oil was the desired ethoxy propyl acrylate product.

## EXAMPLE 7

### 2-Hydroxy-3- 2-(2-Cyano-3,3-Diphenylacryloxy) Ethoxy Propyl Methacrylate

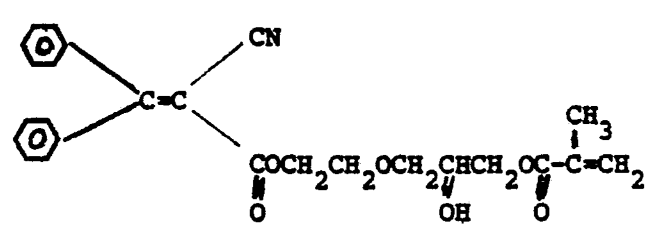

Using glycidyl methacrylate in place of glycidyl acrylate in Example 6 gives the corresponding methacrylate compound.

EXAMPLE 8

2-(2-Cyano-3,3-Diphenylacryloxy) Ethyl Acrylate

(Method 2)

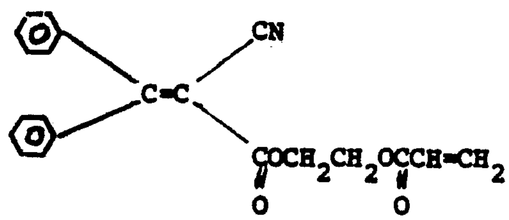

A. Acryloxyethyl 2-Cyanoacetate

Into a flask fitted with a mechanical stirrer and a reflux condenser is charged 1 l. of methylene chloride 129 g. (1 mole), 2-hydroxyethyl 2-cyanoacetate 118 g. (1.3 moles), acryloyl chloride and 179 g. potassium carbonate. The reaction mixture was heated at reflux (41°C) for 2 hours, diluted with 1 l. of water and neutralized with solid potassium carbonate. The organic layer then was separated, dried and evaporated leaving 128 g. (70%) of 2-acryloxyethyl 2-cyanoacetate, which was stabilized with 50 ppm of hydroquinone.

B. In a round bottom flask equipped with a mechanical stirrer, a thermometer, and a Dean-Stark water trap fitted with a reflux condenser was charged 270 ml. toluene, 182 g. (1 mole) of benzophenone, 183 g. (1 mole) of 2-acryloxyethyl 2-cyanoacetate, 26 mg. of phenothiazine, 50 ml. of glacial acetic acid, and 20 g. of ammonium acetate. The solution then was heated at relfux (110°C) for 16 hours. After cooling, the reaction solution was washed with 300 ml. of water and the organic layer was separated and dried. The toluene solvent was evaporated leaving an amber oil which was predominantly the product compound.

## EXAMPLE 9

### Preparation of Radiation Cured Coating

Into a dry 1 l. resin kettle fitted with an air inlet tube, a stirrer, thermometer, and dropping funnel was charged 300.8 g. (1.3 moles) of iosphorone diisocyanate and 4.8 ml. of a 10% (W/V) solution of dibutyltin dilaurate catalyst in ethylhexyacrylate. Dry air then was bubbled through the stirred solution while 322.1 g. (0.61 moles) of polycarpolactone (PCP-200) was added dropwise over 45 minutes. The solution then was heated to 80°C and the reactants maintained at this temperature for 30 minutes. After cooling to 55°C.,160 mg. of phenothiazine was admixed. Then 151.9 g. (1.3 moles) of hydroxyethyl acrylate was added rapidly. The temperature was raised to 80°C and maintained for 2 hours.

The resulting oligomer (58.1 g.) was formulated for coating by mixing with 25.4 g. of ethylhexylacrylate, 16.8 g. of vinyl pyrrolidone, 14.2 g. of hexanediol diacrylate, 1.8 g. of DC-193 silicone surfactant, 2.4 g. of Vicure-10 photo-initiator and 2.5 g. of 2-(2-cyano-3,3-diphenylacryloxy) ethyl acrylate. The resulting syrup was coated onto a polyvinylchloride plate to form a film having a thickness of 1.5 mil. The film then was cured by ultraviolet radiation under an inert atmosphere to provide a tough, clear coating containing the copolymerized UV absorber compound of the invention.

## EXAMPLE 10

### 2-(2-Cyano-3,3-Diphenylacryloxy) Ethyl Allyl Ether

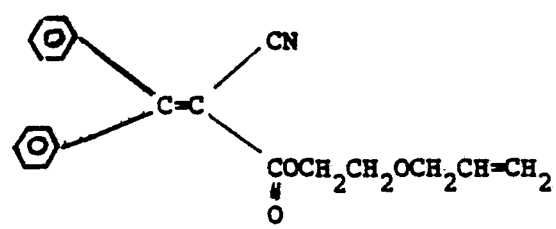

Into a round bottom flask equipped with magnetic stirrer, reflux condenser and drying tube was charged 20 ml. of dry tetrahydrofuran and 3.2 g. (.011 moles) of 2-hydroxyethyl 2-cyano-3,3-diphenylacrylate and the solution is rapidly stirred. Then sodium hydride 0.79 g. (.016 moles) as a 50% oil dispersion was added portionwise. To the resulting suspension was added 5.3 g. (.044 moles) of allyl bromide and the mixture was heated at reflux (66°C) for 5.5 hours. Upon cooling, a white insoluble solid (NaBr) was removed by filtration. The organic filtrate was evaporated, leaving as the desired product a pale yellow oil.

## EXAMPLE 11

## 2-(2-Cyano-3,3-Diphenylacryloxy) Ethyl 2-Butenyl Ether

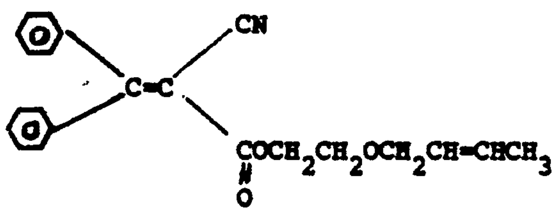

When crotyl bromide was substituted for allyl bromide in Example 10, the desired product is obtained.

## EXAMPLE 12

## 2-(2-Cyano-3,3-Diphenylacryloxy) Ethyl 2-Methyl-2-Propenyl Ether

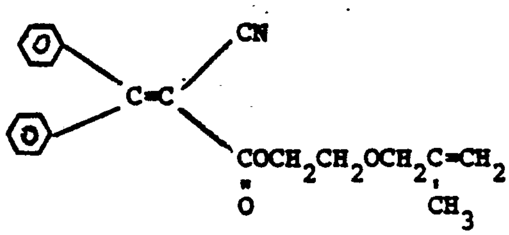

When 3-chloro-2-methyl-1-propene was substituted for allyl bromide in Example 10, the desired product is obtained.

## EXAMPLE 13

2-(2-Cyano-3,3-Diphenylacryloxy) Ethyl Vinylbenzyl Ether

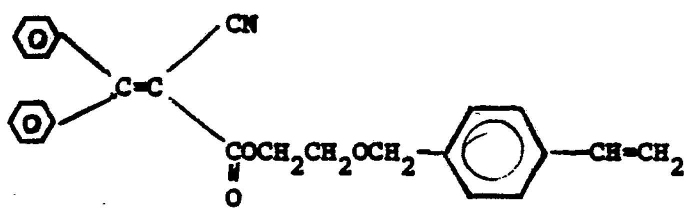

When vinylbenzyl chloride was substituted for allyl bromide in Example 10, the desired product is obtained.

## EXAMPLE 14

2-(2-Cyano-3,3-Diphenylacryloxy) Ethyl Vinyloxyethyl Ether

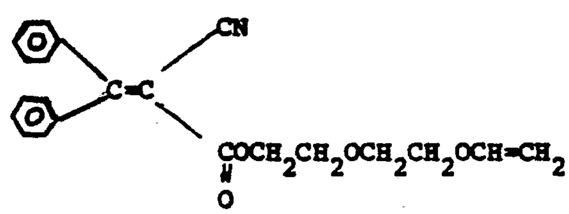

When chloroethylvinyl ether was substituted for allyl bromide in Example 10, the desired product is obtained.

EXAMPLE 15

2-(2-Cyano-3,3-Diphenylacryloxy)-Ethyl-3-Allyloxy-2-Hydroxypropyl Ether

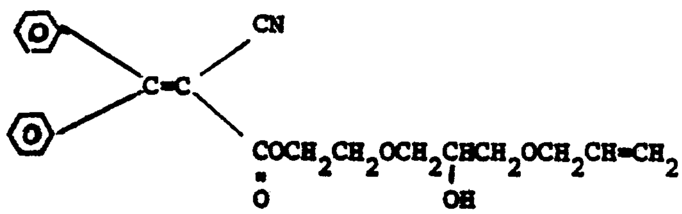

Into a round bottom flask was charged 29 g. (0.1 moles) of 2-hydroxyethyl 2-cyano-3,3-diphenylacrylate, 12.4 g. (0.11 moles) of allyl glycidyl ether and 250 mg. of tetramethylammonium chloride. The flask was stoppered and the mixture was heated at 50°C for 16 hours. Then cooled mixture was diluted with 50 ml. methylene dichloride, treated with decolonizing charcoal and filtered. The filtrate was evaporated, leaving a pale yellow oil characterized as the product.

EXAMPLE 16

2-(2-Cyano-3,3-diphenylacryloxy) Ethyl 2-Hydroxy-3-Butenyl Ether

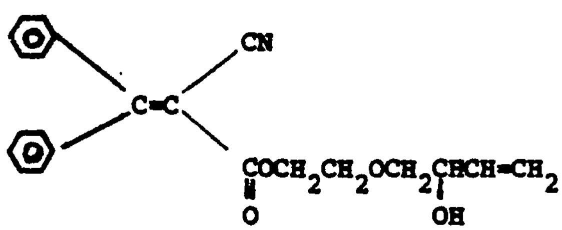

When butadiene monoxide substituted for allyl glycidyl ether in example 15, the desired product was obtained.

EXAMPLE 17

A radiation cured coating as in Example 9 was prepared using the product of Example 10 to provide thereby a though, clear coating.

Further compounds which can be obtained according to the methods described in the above examples are e.g.:
3-(2-cyano-3,3-diphenylacryloxy) propyl acrylate,
3-(2-cyano-3,3-diphenylacryloyloxy)-2-hydroxypropyl acrylate,
3-(2-cyano-3,3-diphenylacryloyloxy)-2-hydroxypropyl methacrylate.

Further objects of the invention are the intermediates:

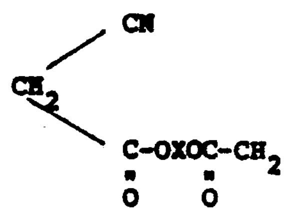

where X is alkylene, $C_2$-$C_{17}$, unsubstituted or substituted with halo, cyano, alkyl, $C_1$-$C_6$, alkoxy, $C_1$-$C_6$, alkoxyalkyl, $C_1$-$C_6$, or alkoxyalkyleneoxy, $C_1$-$C_6$.

and

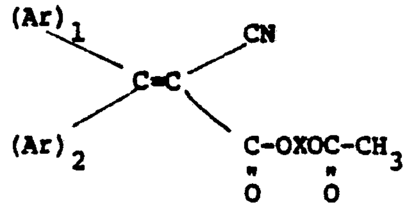

where $(Ar)_1$ and $(Ar)_2$ are aromatic carbocyclic nuclei of the benzene and naphthalene series and are independently selected from phenyl or phenyl substituted with alkyl, halo, alkoxy, carboxy, carbalkoxy, cyano, acetyl, benzoyl, phenyl, alkyl phenyl, phenoxy phenyl, alkyl

substituted phenoxy, or alkoxy phenyl substitued phenyl and naphthyl; and;

X is alkylene, $C_2$-$C_{17}$, unsubstituted or substituted with halo, cyano, alkyl, $C_1$-$C_6$, alkoxy, $C_1$-$C_6$ alkoxyalkyl, $C_1$-$C_6$, or alkoxyalkyleneoxy, $C_1$-$C_6$.

In the above formulae the various residues may e.g. have the following definitions:

All alkyl groups - as far as not stated differently - may contain 1-6, preferably 1-4, carbon atoms. This also concerns complex residues containing alkyl groups, e.g. alkoxy, carbalkoxy, alkylphenyl etc.
This alkyl residues may be: methyl, ethyl, propyl, i-propyl, butyl, i-butyl, sec-butyl, pentyl, i-pentyl, hexyl, i-hexyl, Alkyl groups with longer alkyl chains may additionally be octyl, i-octyl, decyl and i-decyl.

In case of composite residues like alkyloxyalkyl the alkyl parts may have 1-6 carbon atoms each, preferably the sum of all carbon atoms in such residue is, however, 6.

Halogen is F, Cl, Br and I.

WHAT IS CLAIMED IS:

1. Compounds having the formula:

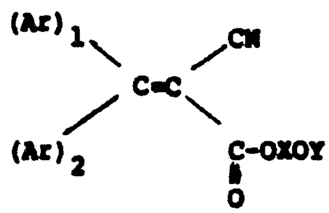

where $(Ar)_1$ and $(Ar)_2$ are aromatic carbocyclic nuclei of the benzene and naphthalene series and are independently selected from phenyl or phenyl substituted with alkyl, halo, alkoxy, carboxy, carbalkoxy, cyano, acetyl, benzoyl, phenyl, alkyl phenyl, phenoxy phenyl, alkyl substituted phenoxy, or alkoxy phenyl substituted phenyl and naphthyl;

X is alkylene, $C_2$-$C_{17}$, unsubstituted or substituted with halo, cyano, alkyl, alkoxy, alkoxyalkyl, or alkoxyalkyleneoxy, $C_1$-$C_6$; and,

Y is a copolymerizable radical selected from acryloyl, alkylacryoyl, acryloxyalkylhydroxyalkyl, $C_3$-$C_{12}$, or -RCR'=CHR", where R is alkylene, oxyalkylene, alkyleneoxyalkylene, phenylene, $C_1$-$C_{10}$, unsubstituted or substituted with hydroxy and R' and R" are independently hydrogen or alkyl, $C_1$-$C_{10}$.

2. A method of making (2-cyano-3,3-diphenylacryloxy) alkylene compounds, having the formula of Claim 1, which comprises:

(a) acrylating a hydroxyalkylene cyanoacetate having the formula:

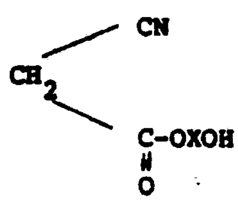

where X is as defined above, with an acylating agent capable of reacting with the hydroxy group to form an acylated intermediate with is convertible to hydroxy by hydrolysis,

(b) condensing the acylated intermediate with a benzophenone having the formula:

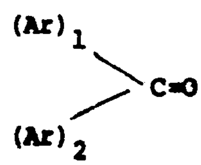

where $(Ar)_1$ and $(Ar)_2$ are as defined above, to form a condensed intermediate;

(c) hydrolyzing the condensed intermediate to form the corresponding condensed hydroxy compound and;

(d) esterifying the condensed hydroxy compound with an acryloyl halide or acrylic acid having the formula:

YZ

where Y is as defined above and Z is a halide or hydroxyl group, or alkylating with:

ZRCR'=CHR"

where Z, R', R" and R are as defined above, to form said compounds.

3. A method of making (2-cyano-3,3-diphenylacryloxy) alkylene compounds in high yield having the formula:

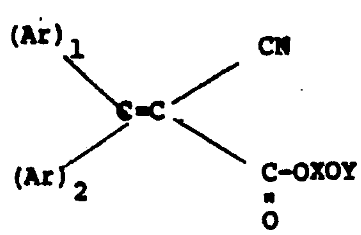

where $(Ar)_1$ and $(Ar)_2$ are aromatic carbocyclic nuclei of the benzene and naphthalene series and are independently selected from phenyl or phenyl substituted with alkyl, halo, alkoxy, carboxy, carbalkoxy, cyano, acetyl, benzoyl, phenyl, alkyl phenyl, phenoxy phenyl, alkyl substituted phenoxy, or alkoxy phenyl substituted phenyl and naphthyl;

X is alkylene, $C_2$-$C_{17}$, unsubstituted or substituted with halo, cyano, alkyl $C_1$-$C_6$, alkoxy $C_1$-$C_6$, alkoxyalkyl $C_1$-$C_6$, or alkoxyalkyleneoxy $C_1$-$C_6$; and,

Y is copolymerizable radical selected from acryloyl $C_3$-$C_{12}$, alkylacryloyl $C_3$-$C_{12}$, acryloxyalkyl $C_3$-$C_{12}$, acryloxyhydroxyalkyl and alkylacryloxyhydroxyalkyl $C_3$-$C_{12}$ which comprises the steps of:

(a) esterifying a hydroxyalkylene cyanoacetate having the formula:

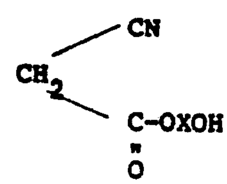

where X is as defined above, with an acryloyl halide or acrylic acid having the formula YZ, where Y is as defined above and Z is a halide or hydroxyl group to form a (2-acryloxyalkyl) 2-cyanoacetate intermediate, and,

(b) condensing the intermediate with a benzophenone having the formula:

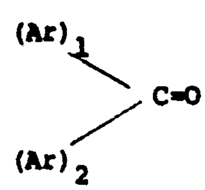

where $(Ar)_1$ and $(Ar)_2$ are as defined above, to form the desired compound.

4. The use of compounds according to claim 1 as UV absorbers.

0017703

European Patent Office

# EUROPEAN SEARCH REPORT

Application number
EP 80100398.9

| Category | Citation of document with indication, where appropriate, of relevant passages | Relevant to claim | CLASSIFICATION OF THE APPLICATION (Int. Cl. 3) |
|---|---|---|---|
| | DE - A - 1 443 923 (GENERAL ANILINE AND FILM CORP.)<br>+ Claims +<br>-- | 1-4 | C 07 C 121/70<br>C 07 C 120/00<br>C 08 F 222/32 |
| D | US - A - 3 644 466 (ALBERT F. STROBEL)<br>+ Claims; column 6, lines 44-50 +<br>-- | 1-4 | |
| | US - A - 3 576 003 (ALBERT F. STROBEL)<br>+ Claim 1 +<br>-- | 1,4 | TECHNICAL FIELDS SEARCHED (Int.Cl. 3) |
| | CH - A - 415 625 (FARBENFABRIKEN BAYER AKTIENGESELLSCHAFT)<br>+ Claims +<br>---- | 1,4 | C 07 C 121/00<br>C 07 C 120/00<br>C 08 F 222/00<br>C 09 D |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 02-04-1980 | HEIN |